# EUROPEAN PATENT SPECIFICATION

(11) **EP 1 079 865 B1**
(45) Date of publication and mention of the grant of the patent: **20.10.2004**
(21) Application number: 99918140.7
(22) Date of filing: 22.04.1999
(51) Int. Cl.: A61K 49/00

(54) **ULTRASOUND CONTRAST AGENT DISPERSIONS COMPRISING GAS AND DESTABILISING AGENT**
GAS UND DESTABILISIERUNGSMITTEL ENTHALTENDE ULTRASCHALLKONTRASTMITTELDISPERSONEN
DISPERSIONS AVEC AGENT DE CONTRASTE AUX ULTRASONS RENFERMANT UN GAZ ET UN AGENT DESTABILISANT

(30) Priority: 22.04.1998 GB 9808582
(43) Date of publication of application: 07.03.2001
(73) Proprietor: Amersham Health AS, 0401 Oslo (NO)
(72) Inventor: SKURTVEIT, Roald, Amersham Health AS, Nydalen, 0401 Oslo (NO); HJELSTUEN, Olaug, Amersham Health AS, Nydalen, 0401 Oslo (NO); OSTENSEN, Jonny, Amersham Health AS, Nydalen, 0401 Oslo (NO)
(74) Representative: Canning, Lewis R.
(86) International application number: PCT/GB1999/001228
(87) International publication number: WO 1999/053964

(56) References cited:
- EP-A- 0 212 568
- EP-A- 0 365 467
- WO-A-95/07072
- WO-A-98/10799
- WO-A-98/17324
- US-A- 5 685 310
- WEI K ET AL: "Quantification of Myocardial Blood Flow With Ultrasound-Induced Destruction of Microbubbles Administered as a Constant Venous Infusion" CIRCULATION, vol. 5, no. 97, 10 February 1998 (1998-02-10), pages 473-483, XP002077428 ISSN: 0009-7322
- WEY K ET AL: "Use of Microbubble Destruction as a Novel Approach for Quantification of Myocardial Perfusion With Contrast Echocardiography During Venous Infusion of Contrast" JOURNAL OF THE AMERICAN COLLEGE OF CARDIOLOGY,1 February 1997 (1997-02-01), XP002077429 ISSN: 0735-1097
- VANDENBERG B. ET AL.: "Myocardial risk area and peak gray level measurement by contrast echocardiography: Effect of microbubble size and concentration, injection rate, and coronary vasodilation" AMERICAN HEART JOURNAL, vol. 115, 1988, page 733-739 XP002112966

## Description

This invention relates to ultrasound imaging, more particularly to novel contrast agent preparations and their use in ultrasound imaging, for example in visualising tissue perfusion.

It is well known that contrast agents comprising dispersions of microbubbles of gases are particularly efficient backscatterers of ultrasound by virtue of the low density and ease of compressibility of the microbubbles. Such microbubble dispersions, if appropriately stabilised, may permit highly effective ultrasound visualisation of, for example, the vascular system and tissue microvasculature, often at advantageously low doses.

The use of ultrasonography to measure blood perfusion (i.e. blood flow per unit of tissue mass) is of potential value in, for example, tumour detection, tumour tissue typically having different vascularity from healthy tissue, and studies of the myocardium, e.g. to detect myocardial infarctions. A problem with the application of existing ultrasound contrast agents to cardiac perfusion studies is that the information content of images obtained is degraded by attenuation caused by contrast agent present in the ventricles of the heart.

In our copending International Patent Publication No. WO-A-9817324 we have disclosed that ultrasonic visualisation of a subject, in particular of perfusion in the myocardium and other tissues, may be achieved and/or enhanced by means of gas-containing contrast agent preparations which promote controllable and temporary growth of the gas phase *in vivo* following administration. Such contrast agent preparations may be used to promote controllable and temporary retention of the gas phase, for example in the form of microbubbles, in tissue microvasculature, thereby enhancing the concentration of gas in such tissue and accordingly enhancing its echogenicity, e.g. relative to the blood pool.

Such use of gas as a deposited perfusion tracer differs markedly from existing proposals regarding intravenously administrable microbubble ultrasound contrast agents. Thus it is generally thought necessary to avoid microbubble growth since, if uncontrolled, this may lead to potentially hazardous tissue embolisation. Accordingly it may be necessary to limit the dose administered and/or to use gas mixtures with compositions selected so as to minimise bubble growth *in vivo* by inhibiting inward diffusion of blood gases into the microbubbles (see e.g. WO-A-9503835 and WO-A-9516467).

In accordance with WO-A-9817324, on the other hand, a composition comprising a dispersed gas phase is coadministered with a composition comprising at least one substance which has or is capable of generating a gas or vapour pressure *in viv*o sufficient to promote controllable growth of the said dispersed gas phase through inward diffusion thereto of molecules of gas or vapour derived from said substance. This latter substance is referred to as a "diffusible component", although it will be appreciated that transport mechanisms other than diffusion may additionally or alternatively be involved.

The preparations may advantageously be employed in visualising tissue perfusion in a subject, the increase in size of the dispersed gas being utilised to effect enrichment or temporary retention of gas in the microvasculature of such tissue, thereby enhancing its echogenicity.

The dispersed gas content of such contrast agent preparations will tend to be temporarily retained in tissue in concentrations proportional to the regional rate of tissue perfusion. Accordingly, when using ultrasound imaging modalities such as conventional or harmonic B-mode imaging where the display is derived directly from return signal intensities, images of such tissue may be interpreted as perfusion maps in which the displayed signal intensity is a function of local perfusion. This is in contrast to images obtained using free-flowing contrast agents, where the regional concentration of contrast agent and corresponding return signal intensity depend on the actual blood content rather than the rate of perfusion of local tissue.

The present invention is based on the finding that growth of a dispersed gas in order to bring about its temporary retention in tissue microvasculature may be effected by administering the gas dispersion in conjunction with one or more substances serving to destabilise the dispersion. By avoiding use of a diffusible component requiring transport through blood and/or the continuous phase(s) in which the gas dispersion and diffusible component are contained, as well as through any encapsulating or other stabilising membranes which surround them, the invention may allow greater control of growth of the dispersed gas and may also involve a higher proportion of gas-containing structures in the growth process.

Thus according to one aspect of the present invention there is provided a combined preparation for simultaneous, separate or sequential use as a contrast agent in ultrasound imaging, said preparation comprising:
i) an injectable aqueous gas dispersion, and
ii) a separately administrable substance or substances capable of destabilising said dispersed gas so as at least transiently to increase the size thereof.

According to a further aspect of the invention there is provided a method of generating enhanced images of a human or non-human animal subject which comprises the steps of:
i) injecting a physiologically acceptable aqueous medium having gas dispersed therein into the vascular system of said subject;
ii) before, during or after injection of said aqueous medium administering to said subject a substance or substances capable of destabilising said dispersed gas so as at least transiently to increase the size thereof; and
iii) generating an ultrasound image of at least a part of said subject.

In general any biocompatible gas may be present in the gas dispersion used in accordance with the invention, the term "gas" as used herein including any substances (including mixtures) at least partially, e.g. substantially or completely, in gaseous or vapour form at the normal human body temperature of 37°C. Representative gases thus include air; nitrogen; oxygen; carbon dioxide; hydrogen; inert gases such as helium, argon, xenon or krypton; sulphur fluorides such as sulphur hexafluoride, disulphur decafluoride or trifluoromethylsulphur pentafluoride; selenium hexafluoride; optionally halogenated silanes such as methylsilane or dimethylsilane; low molecular weight hydrocarbons (e.g. containing up to 7 carbon atoms), for example alkanes such as methane, ethane, a propane, a butane or a pentane, cycloalkanes such as cyclopropane, cyclobutane or cyclopentane, alkenes such as ethylene, propene, propadiene or a butene, or alkynes such as acetylene or propyne; ethers such as dimethyl ether; ketones; esters; halogenated low molecular weight hydrocarbons (e.g. containing up to 7 carbon atoms); or mixtures of any of the foregoing. Advantageously at least some of the halogen atoms in halogenated gases are fluorine atoms; thus biocompatible halogenated hydrocarbon gases may, for example, be selected from bromochlorodifluoromethane, chlorodifluoromethane, dichlorodifluoromethane, bromotrifluoromethane, chlorotrifluoromethane, chloropentafluoroethane, dichlorotetrafluoroethane, chlorotrifluoroethylene, fluoroethylene, ethylfluoride, 1,1-difluoroethane and perfluorocarbons. Representative perfluorocarbons include perfluoroalkanes such as perfluoromethane, perfluoroethane, perfluoropropanes or perfluorobutanes (e.g. perfluoro-n-butane, optionally in admixture with other isomers such as perfluoro-iso-butane); perfluoroalkenes such as perfluoropropene, perfluorobutenes (e.g. perfluorobut-2-ene), perfluorobutadiene, perfluoropentenes (e.g. perfluoropent-1-ene) or perfluoro-4-methylpent-2-ene; perfluoroalkynes such as perfluorobut-2-yne; and perfluorocycloalkanes such as perfluorocyclobutane, perfluoromethylcyclobutane, perfluorodimethylcyclobutanes, perfluorotrimethylcyclobutanes, perfluorocyclopentane, perfluoromethylcyclopentane, perfluorodimethylcyclopentanes, perfluorocyclohexane, perfluoromethylcyclohexane or perfluorocycloheptane. Other halogenated gases include methyl chloride, fluorinated (e.g. perfluorinated) ketones such as perfluoroacetone and fluorinated (e.g. perfluorinated) ethers such as perfluorodiethyl ether. The use of perfluorinated gases, for example sulphur hexafluoride and perfluorocarbons such as perfluoropropane, perfluorobutanes, perfluoropentanes and perfluorohexanes, may be particularly advantageous in view of the recognised high stability in the bloodstream of microbubbles containing such gases. Other gases with physicochemical characteristics which cause them to form highly stable microbubbles in the bloodstream may likewise be useful.

The dispersed gas may, for example, be present in the form of microbubbles at least partially encapsulated or otherwise stabilised by gas-stabilising material. This stabilising material may, for example, comprise an initially coalescence-resisting surface membrane (for example gelatin, e.g. as described in WO-A-8002365), a filmogenic protein (for example an albumin such as human serum albumin, e.g. as described in US-A-4718433, US-A-4774958, US-A-4844882, EP-A-0359246, WO-A-9112823, WO-A-9205806, WO-A-9217213, WO-A-9406477 or WO-A-9501187), a polymer material (for example a synthetic biodegradable polymer as described in EP-A-0398935, an elastic interfacial synthetic polymer membrane as described in EP-A-0458745, a microparticulate biodegradable polyaldehyde as described in EP-A-0441468, a microparticulate N-dicarboxylic acid derivative of a polyamino acid - polycyclic imide as described in EP-A-0458079, or a biodegradable polymer as described in WO-A-9317718 or WO-A-9607434), a non-polymeric and non-polymerisable wall-forming material (for example as described in WO-A-9521631), or a surfactant (for example a polyoxyethylene-polyoxypropylene block copolymer surfactant such as a Pluronic, a polymer surfactant as described in WO-A-9506518, or a film-forming surfactant such as a phospholipid, e.g. as described in WO-A-9211873, WO-A-9217212, WO-A-9222247, WO-A-9428780, WO-A-9503835 or WO-A-9729783).

Where phospholipid-containing gas dispersions are employed in accordance with the invention, e.g. in the form of phospholipid-stabilised gas microbubbles, representative examples of useful phospholipids include lecithins (i.e. phosphatidylcholines), for example natural lecithins such as egg yolk lecithin or soya bean lecithin, semisynthetic (e.g. partially or fully hydrogenated) lecithins and synthetic lecithins such as dimyristoylphosphatidylcholine, dipalmitoylphosphatidylcholine or distearoylphosphatidylcholine; phosphatidic acids; phosphatidylethanolamines; phosphatidylserines; phosphatidylglycerols; phosphatidylinositols; cardiolipins; sphingomyelins; fluorinated analogues of any of the foregoing; mixtures of any of the foregoing and mixtures with other lipids such as cholesterol. The use of phospholipids predominantly (e.g. at least 75%) comprising molecules individually bearing net overall charge, for example as described in WO-A-9729783, may be particularly advantageous. Representative negatively charged phospholipids include naturally occurring (e.g. soya bean or egg yolk derived), semisynthetic (e.g. partially or fully hydrogenated) and synthetic phosphatidylserines, phosphatidylglycerols, phosphatidylinositols, phosphatidic acids and/or cardiolipins. Representative positively charged phospholipids include esters of phosphatidic acids such as dipalmitoylphosphatidic acid or distearoylphosphatidic acid with aminoalcohols such as hydroxyethylenediamine.

Gas microbubbles preferably have an initial average size not exceeding 10 µm (e.g. of 7 µm or less) in order to permit their free passage through the pulmonary system following administration, e.g. by intravenous injection. However, larger microbubbles may be employed where, for example, these contain a mixture of one or more relatively blood-soluble or otherwise diffusible gases such as air, oxygen, nitrogen or carbon dioxide with one or more substantially insoluble and nondiffusible gases such as perfluorocarbons. Outward diffusion of the soluble/diffusible gas content following administration will cause such microbubbles rapidly to shrink to a size which will be determined by the amount of insoluble/non-diffusible gas present and which may be selected to permit passage of the resulting microbubbles through the lung capillaries of the pulmonary system.

The destabilising substance(s) (hereinafter referred to as "the destabiliser") employed to induce at least transient growth of the dispersed gas phase may, for example, operate by mechanisms involving (i) flocculation, aggregation or agglomeration whereby dispersed gas microbubbles assemble together to form clusters; (ii) coalescence or fusion of the dispersed gas moieties whereby two or more such moieties merge to create a larger moiety; or (iii) Ostwald ripening of dispersed gas moieties, where larger microbubbles grow at the expense of smaller ones, the driving force being the higher pressure in small microbubbles compared to large microbubbles as a result of differences in surface tension in accordance with Laplace's law. It will be appreciated that two or more mechanisms may operate simultaneously.

Flocculation, aggregation or agglomeration may be achieved by making the interbubble repulsive forces weaker than the interbubble attractive forces. The size of the resulting clusters may depend on factors such as the initial size and concentration of the microbubbles, the hydrodynamic behaviour of the surrounding blood stream, the ambient temperature and the net attractive force between the microbubbles.

Where the dispersed gas is stabilised by charge interactions, for example by interactions between charged surface groups in stabilising membranes of materials such as charged phospholipids, addition of charged species such as electrolytes will supply counter-ions which will screen the charged surface groups and reduce interbubble repulsive forces. The use of di- and multi-valent counter-ions is preferred; thus, for example, salts, e.g. inorganic salts such as calcium chloride or magnesium chloride may be used to destabilise negatively charged membranes.

Aggregation may also be induced by modifying the properties of the surrounding water phase, for example by addition of solvents or of substances which change the electrical permeability of the medium so as to destabilise charge-stabilised gas dispersions. Representative examples of such substances include water-miscible liquids or solids, e.g. aliphatic alcohols such as ethanol, isopropanol, ethylene glycol, propylene glycol, glycerol and sorbitol; aliphatic aldehydes and ketones such as acetaldehyde and acetone; aliphatic esters such as methyl acetate, propyl formate and ethyl acetate; aliphatic ethers such as methyl propyl ether and di-isopropyl ether; aliphatic amides such as N,N-dimethylformamide and N,N-dimethylacetamide; aliphatic nitriles such as acetonitrile; carbohydrates such as glucose and sucrose; polyethylene glycol; polypropylene glycol etc. Aliphatic compounds as described herein typically contain up to 6 carbon atoms.

Other representative destabilisers include components capable of bridging dispersed gas microbubbles. Examples of such components include polymers, e.g. polysaccharides such as dextran and starches; polyaminoacids such as polylysine and proteins such as gelatin and albumin; polyethers such as polyethylene glycol, polypropylene glycol and polyoxyethylene-polyoxypropylene block copolymers such as Pluronics; polyvinyl pyrrolidone etc.

Aggregation may also be induced by adding components which enter and modify the membrane, thereby altering the forces between different microbubbles. Examples of such compounds include fatty alcohols, fatty acids and fatty amines; various surfactants; steroids such as cholesterol etc.

Where the gas-stabilising material contains acid or base groups on its surface, destabilisation may be effected by addition of an appropriate acidic or basic destabiliser in order to change the pH.

Aggregation may be a reversible or irreversible process, depending on the magnitude of the forces between the dispersed gas moieties. By optimising these forces the aggregates may be made loose enough to be easily disintegrated a short time after their temporary retention in tissue microvasculature, thus ensuring that the imaging procedure has a high safety profile. Such disintegration or redispersion of the aggregates may if desired be induced or enhanced by subsequent administration of an appropriate substance. For instance, a complexing agent such as ethylenediamine tetraacetic acid may remove or neutralise the effect of divalent or polyvalent ions used to destabilise charged microbubbles. A buffer, acid or base may be used to remove the effect of pH-induced aggregation/destabilistation. Solvents, e.g. alcohols such as ethanol, isopropanol, ethylene glycol, propylene glycol and glycerol; aldehydes and ketones such as acetaldehyde and acetone; ethers such as methyl propyl ether and di-isopropyl ether; aliphatic amides such as N,N-dimethylformamide and N,N-dimethylacetamide; or aliphatic nitriles such as acetonitrile, may for example be added to change the bridging behaviour of a polymeric flocculant by altering the polymer's ability to coil in the solvent. Such substances therefore re-establish the repulsive forces between the dispersed gas moieties.

Destabilisers which operate by inducing coalescence or fusion of the dispersed gas moieties include substances which modify the properties of gas-stabilising membrane material so that membrane rupture between coalescing gas moieties is enhanced. Representative destabilisers of this type include surfactants, co-surfactants, solvents and other substances which penetrate or otherwise modify the properties of the stabilising membrane. Chemical modification of the stabilising membrane, e.g. by addition of electrolytes or by pH change, or chemical reaction within the stabilising membrane may likewise be used to induce flocculation.

Growth of dispersed gas moieties by Ostwald ripening may be initiated by increasing the solubility of the gas in the surrounding aqueous phase, for example by adding a solvent or other appropriate component, e.g. a hydrotrope such as sodium benzoate or acetic acid, or a surfactant capable of solubilising the gas within micelles. Representative examples of solvents which may increase the solubility of the gas in the surrounding aqueous phase include aliphatic ethers such as ethyl methyl ether and methyl propyl ether; aliphatic esters such as methyl acetate, methyl formate and ethyl formate; aliphatic ketones such as acetone; aliphatic amides such as N,N-dimethylformamide and N,N-dimethylacetamide; and aliphatic nitriles such as acetonitrile. Representative examples of surfactants which may increase the solubility of the gas in the surrounding aqueous phase are those forming micelles in water, e.g. alkyl carboxylates, alkyl sulphonates, alkyl sulphates, dialkyl sulphosuccinates, alkylpyridinium salts, alkylammonium salts, alkyl polyethylene glycol ethers, alkyl polyethylene glycol esters and sorbitol fatty acid esters; alkyl groups in such surfactants may, for example, contain 5-30 carbon atoms.

Ostwald ripening kinetics may also be enhanced by increasing the interfacial tension about the dispersed gas, for example by modifying the properties of stabilising membranes, e.g. through solvent addition, salt addition, pH change or addition of surfactants/ co-surfactants which provide higher surface tension.

As noted above, the destabiliser may be administered before, during or after injection of the gas dispersion; the order of administration and timing between them when they are separate may be varied to permit control over the time, rate and thus location of temporary retention of the dispersed gas.

In many instances the destabiliser will be administered by injection, e.g. intravenously, although intramuscular or subcutaneous injections may be useful in order to localise the effect of the destabiliser. Volatile destabilisers, e.g. solvents such as ethanol, may, however, also be administered by inhalation; this may be of advantage in avoiding unwanted growth of the dispersed gas in the lung capillaries.

Imaging modalities which may be used in accordance with the invention include two- and three-dimensional imaging techniques such as B-mode imaging (for example using the time-varying amplitude of the signal envelope generated from the fundamental frequency of the emitted ultrasound pulse, from sub-harmonics or higher harmonics thereof or from sum or difference frequencies derived from the emitted pulse and such harmonics, images generated from the fundamental frequency or the second harmonic thereof being preferred), colour Doppler imaging, Doppler amplitude imaging and combinations of these last two techniques with any of the other modalities described above. To reduce the effects of movement, successive images of tissues such as the heart or kidney may be collected with the aid of suitable synchronisation techniques (e.g. gating to the ECG or respiratory movement of the subject). Measurement of changes in resonance frequency or frequency absorption which accompany growth of the dispersed gas may also usefully be made to detect the contrast agent.

It will be appreciated that the dispersed gas content of combined contrast agent preparations according to the invention will, following destabilisation, tend to be temporarily retained in tissue in concentrations proportional to the regional rate of tissue perfusion. Accordingly, when using ultrasound imaging modalities such as conventional or harmonic B-mode imaging where the display is derived directly from return signal intensities, images of such tissue may be interpreted as perfusion maps in which the displayed signal intensity is a function of local perfusion. This is in contrast to images obtained using free-flowing contrast agents, where the regional concentration of contrast agent and corresponding return signal intensity depend on the actual blood content rather than the rate of perfusion of local tissue.

In cardiac studies, where perfusion maps are derived from return signal intensities in accordance with this embodiment of the invention, it may be advantageous to subject a patient to physical or pharmacological stress in order to enhance the distinction, and thus the difference in image intensities, between normally perfused myocardium and any myocardial regions supplied by stenotic arteries. As is known from radionuclide cardiac imaging, such stress induces vasodilatation and increased blood flow in healthy myocardial tissue, whereas blood flow in underperfused tissue supplied by a stenotic artery is substantially unchanged since the capacity for arteriolar vasodilatation is already exhausted by inherent autoregulation seeking to increase the restricted blood flow.

The application of stress as physical exercise or pharmacologically by administration of adrenergic agonists may cause discomfort such as chest pains in patient groups potentially suffering from heart disease, and it is therefore preferable to enhance the perfusion of healthy tissue by administration of a vasodilating drug, for example selected from adenosine, dipyridamole, nitroglycerine, isosorbide mononitrate, prazosin, doxazosin, dihydralazine, hydralazine, sodium nitroprusside, pentoxyphylline, amelodipine, felodipine, isradipine, nifedipine, nimodipine, verapamil, diltiazem and nitrous oxide. In the case of adenosine this may lead to in excess of fourfold increases in coronary blood flow in healthy myocardial tissue, greatly increasing the uptake and temporary retention of contrast agents in accordance with the invention and thus significantly increasing the difference in return signal intensities between normal and hypoperfused myocardial tissue. Because an essentially physical entrapment process is involved, retention of contrast agents according to the invention is highly efficient; this may be compared to the uptake of radionucleide tracers such as thallium 201 and technetium sestamibi, which is limited by low contact time between tracer and tissue and so may require maintenance of vasodilatation for the whole period of blood pool distribution for the tracer (e.g. 4-6 minutes for thallium scintigraphy) to ensure optimum effect. The contrast agents of the invention, on the other hand, do not suffer such diffusion or transport limitations, and since their retention in myocardial tissue may also rapidly be terminated by the methods described above, the period of vasodilatation needed to achieve cardiac perfusion imaging in accordance with this embodiment of the invention may be very short, for example less than one minute. This will reduce the duration of any possible discomfort caused to patients by administration of vasodilator drugs.

In view of the fact that the required vasodilatation need only be short lasting, adenosine is a particularly useful vasodilating drug, being both an endogenous substance and having a very short-lasting action as evidenced by a blood pool half-life of only 2 seconds. Vasodilatation will accordingly be most intense in the heart, since the drug will tend to reach more distal tissues in less than pharmacologically active concentrations. It will be appreciated that because of this short half-life, repeated injection or infusion of adenosine may be necessary during cardiac imaging in accordance with this embodiment of the invention; by way of example, an initial administration of 150 µg/kg of adenosine may be made substantially simultaneously with administration of the contrast agent composition, followed 10 seconds later by slow injection of a further 150 µg/kg of adenosine, e.g. over a period of 20 seconds.

Contrast agent preparations in accordance with the invention may advantageously be employed as delivery agents for bioactive moieties such as therapeutic drugs (i.e. agents having a beneficial effect on a specific disease in a living human or non-human animal), particularly to targeted sites. Thus, for example, therapeutic compounds may be present in the dispersed gas or may be linked to part of the stabilising material, e.g. through covalent or ionic bonds, if desired through a spacer arm.

The controllable growth properties of the dispersed gas may be utilised to bring about its temporary retention in the microvasculature of a target region of interest, and thus retention of the gas and associated therapeutic compound in a target structure is particularly advantageous.

The therapeutic compound, which may if desired be coupled to a site-specific vector having affinity for specific cells, structures or pathological sites, may be released as a result of, for example, stretching or fracture of the gas-stabilising material caused by growth of the dispersed gas, solubilisation of the stabilising material, or disintegration of gas-microbubbles or gas-containing microparticles. Where a therapeutic agent is chemically linked to the gas-stabilising material, the linkage or any spacer arm associated therewith may advantageously contain one or more labile groups which are cleavable to release the agent. Representative cleavable groups include amide, imide, imine, ester, anhydride, acetal, carbamate, carbonate, carbonate ester and disulphide groups which are biodegradable *in vivo,* e.g. as a result or hydrolytic and/or enzymatic action.

Representative and non-limiting examples of drugs useful in accordance with this embodiment of the invention include antineoplastic agents such as vincristine, vinblastine, vindesine, busulfan, chlorambucil, spiroplatin, cisplatin, carboplatin, methotrexate, adriamycin, mitomycin, bleomycin, cytosine arabinoside, arabinosyl adenine, mercaptopurine, mitotane, procarbazine, dactinomycin (antinomycin D), daunorubicin, doxorubicin hydrochloride, taxol, plicamycin, aminoglutethimide, estramustine, flutamide, leuprolide, megestrol acetate, tamoxifen, testolactone, trilostane, amsacrine (m-AMSA), asparaginase (L-asparaginase), etoposide, interferon a-2a and 2b, blood products such as hematoporphyrins or derivatives of the foregoing; biological response modifiers such as muramylpeptides; antifungal agents such as ketoconazole, nystatin, griseofulvin, flucytosine, miconazole or amphotericin B; hormones or hormone analogues such as growth hormone, melanocyte stimulating hormone, estradiol, beclomethasone dipropionate, betamethasone, cortisone acetate, dexamethasone, flunisolide, hydrocortisone, methylprednisolone, paramethasone acetate, prednisolone, prednisone, triamcinolone or fludrocortisone acetate; vitamins such as cyanocobalamin or retinoids; enzymes such as alkaline phosphatase or manganese superoxide dismutase; antiallergic agents such as amelexanox; anticoagulation agents such as warfarin, phenprocoumon or heparin; antithrombotic agents; circulatory drugs such as propranolol; metabolic potentiators such as glutathione; antituberculars such as p-aminosalicylic acid, isoniazid, capreomycin sulfate, cyclosexine, ethambutol, ethionamide, pyrazinamide, rifampin or streptomycin sulphate; antivirals such as acyclovir, amantadine, azidothymidine, ribavirin or vidarabine; blood vessel dilating agents such as diltiazem, nifedipine, verapamil, erythritol tetranitrate, isosorbide dinitrate, nitroglycerin or pentaerythritol tetranitrate; antibiotics such as dapsone, chloramphenicol, neomycin, cefaclor, cefadroxil, cephalexin, cephradine, erythromycin, clindamycin, lincomycin, amoxicillin, ampicillin, bacampicillin, carbenicillin, dicloxacillin, cyclacillin, picloxacillin, hetacillin, methicillin, nafcillin, penicillin or tetracycline; antiinflammatories such as diflunisal, ibuprofen, indomethacin, meclefenamate, mefenamic acid, naproxen, phenylbutazone, piroxicam, tolmetin, aspirin or salicylates; antiprotozoans such as chloroquine, metronidazole, quinine or meglumine antimonate; antirheumatics such as penicillamine; narcotics such as paregoric; opiates such as codeine, morphine or opium; cardiac glycosides such as deslaneside, digitoxin, digoxin, digitalin or digitalis; neuromuscular blockers such as atracurium mesylate, gallamine triethiodide, hexafluorenium bromide, metocurine iodide, pancuronium bromide, succinylcholine chloride, tubocurarine chloride or vecuronium bromide; sedatives such as amobarbital, amobarbital sodium, apropbarbital, butabarbital sodium, chloral hydrate, ethchlorvynol, ethinamate, flurazepam hydrochloride,. glutethimide, methotrimeprazine hydrochloride, methyprylon, midazolam hydrochloride, paraldehyde, pentobarbital, secobarbital sodium, talbutal, temazepam or triazolam; local anaesthetics such as bupivacaine, chloroprocaine, etidocaine, lidocaine, mepivacaine, procaine or tetracaine; general anaesthetics such as droperidol, etomidate, fentanyl citrate with droperidol, ketamine hydrochloride, methohexital sodium or thiopental and pharmaceutically acceptable salts (e.g. acid addition salts such as the hydrochloride or hydrobromide or base salts such as sodium, calcium or magnesium salts) or derivatives (e.g. acetates) thereof; and radiochemicals, e.g. comprising beta-emitters. Of particular importance are antithrombotic agents such as vitamin K antagonists, heparin and agents with heparin-like activity such as antithrombin III, dalteparin and enoxaparin; blood platelet aggregation inhibitors such as ticlopidine, aspirin, dipyridamole, iloprost and abciximab; and thrombolytic enzymes such as streptokinase and plasminogen activator. Other examples of therapeutics include genetic material such as nucleic acids, RNA, and DNA of natural or synthetic origin, including recombinant RNA and DNA. DNA encoding certain proteins may be used in the treatment of many different types of diseases. For example, tumour necrosis factor or interleukin-2 may be provided to treat advanced cancers; thymidine kinase may be provided to treat ovarian cancer or brain tumors; interleukin-2 may be provided to treat neuroblastoma, malignant melanoma or kidney cancer; and interleukin-4 may be provided to treat cancer.

Contrast agent preparations in accordance with the invention may be used as vehicles for contrast-enhancing moieties for imaging modalities other than ultrasound, for example X-ray, light imaging, magnetic resonance and, more preferably, scintigraphic imaging agents. Controlled growth of the dispersed gas phase may be used to position such agents in areas of interest within the bodies of subjects, which may then be imaged.

Contrast agent preparations in accordance with the invention may also be used as vehicles for therapeutically active substances which do not necessarily require release from the preparation in order to exhibit their therapeutic effect. Such preparations may, for example, incorporate radioactive atoms or ions such as beta-emitters which exhibit a localised radiation-emitting effect following growth of the dispersed gas phase and temporary retention of the agent at a target site. It will be appreciated that such agents should preferably be designed so that subsequent shrinkage and cessation of retention of the dispersed gas does not occur until the desired therapeutic radiation dosage has been administered.

Contrast agent preparations in accordance with the invention may additionally exhibit therapeutic properties in their own right. Thus, for example, the dispersed gas may be targeted to capillaries leading to tumours and may act as cell toxic agents by blocking such capillaries. Concentrations of dispersed gas in capillaries may also enhance absorption of ultrasonic energy in hyperthermic therapy; this may be used in, for example, treatment of liver tumours. Irradiation with a relatively high energy (e.g. 5 W) focused ultrasound beam, e.g. at 1.5 MHz, may be appropriate in such applications.

The following non-limitative Examples serve to illustrate the invention.

### Preparation 1

### Phosphatidylserine-stabilised perfluorobutane microbubble dispersion

Hydrogenated phosphatidylserine (5 mg/ml in a 1% w/w solution of propylene glycol in purified water) and perfluorobutane gas were homogenised in-line at 7800 rpm and ca. 40°C to yield a creamy-white microbubble dispersion. The dispersion was fractionated to substantially remove undersized microbubbles (≺2 µm) and the volume of the dispersion was adjusted to the desired microbubble concentration by adding aqueous sucrose to give a sucrose concentration of 92 mg/ml. 2 ml portions of the resulting dispersion were filled into 10 ml flat-bottomed vials specially designed for lyophilisation, and the contents were lyophilised to give a white porous cake. The lyophilisation chamber was then filled with perfluorobutane and the vials were sealed. Prior to use, water was added to a vial and the contents were gently hand-shaken for several seconds to give a perfluorobutane microbubble dispersion.

### Preparation 2

### Phosphatidylserine-stabilised perfluorobutane microbubble dispersion

Hydrogenated egg phosphatidylserine (50 mg) was dispersed in water (9.75 ml, reversed osmosis quality) containing propylene glycol (0.25 ml) by heating to 80°C for about 2 minutes, followed by cooling to room temperature. 1 ml portions of the dispersion were transferred into ten 2 ml chromatography vials. The headspaces were flushed with perfluorobutane gas and the vials were capped and shaken on an Espe CapMix® shaker for 45 seconds, yielding samples of a milky white microbubble dispersion. The microbubble dispersions were collected in two 7 ml vials which were capped and placed on a roller table for two days. Later the samples were stored standing at room temperature until use.

### Preparation 3

### Sodium dodecyl sulphate solution

Sodium dodecyl sulphate (2.09 g) was added to water (7 ml, reversed osmosis quality) at room temperature to obtain a clear solution.

### Preparation 4

### Brij 99 solution

Brij 99 (2.84 g) was added to water (9.5 ml, reversed osmosis quality) and placed on a roller table overnight. A clear, slightly viscous solution was obtained.

### Preparation 5

### Sodium dodecyl sulphate/n-butanol/water microemulsion

Sodium dodecyl sulphate (12.5810 g), n-butanol (27.0813 g) and water (17.07 g, reversed osmosis quality) were mixed and diluted further with water to a water content of 30% w/w, yielding a clear, transparent microemulsion.

### EXAMPLE 1

### Microbubble aggregation by calcium chloride

One drop of the microbubble dispersion prepared in Preparation 1 was placed on an object glass for microscopy investigation. The sample was covered with a cover glass and placed under a microscope. Droplets of a 50 mg/ml calcium chloride solution in water were added to the edge of the cover glass so that the solution penetrated into the microbubble dispersion. The behaviour of the microbubble dispersion as the calcium chloride solution front moved was recorded on a video tape. Microbubble aggregates with larger dimensions than the initial microbubbles were observed to form, demonstrating that microbubbles with potential for retention in capillary systems were generated.

### EXAMPLE 2

### Microbubble aggregation by sodium dodecyl sulphate

One drop of the microbubble dispersion prepared in Preparation 2 was placed on an object glass and covered by a cover glass. The sample was placed under a Zeiss Axioskop optical microscope equipped with a 10 x objective and a camera connected to a computer and an image analysis system. A droplet of the sodium dodecyl sulphate solution from Preparation 3 was placed on the edge of the coverglass, so that the solution penetrated under the coverglass and into the microbubble dispersion. The behaviour of the microbubble dispersion as the sodium dodecyl sulphate solution front moved was recorded, showing formation of microbubble aggregates with larger dimensions than the initial microbubbles, demonstrating the formation of microbubbles with potential for retention in capillary systems.

### EXAMPLE 3

### Microbubble aggregation by Brij 99

The procedure described in Example 2 was repeated using the same microbubble dispersion from Preparation 2 but adding the Brij 99 surfactant solution from Preparation 4 to the edge of the coverglass. The experiment showed formation of microbubble aggregates with larger dimensions than the initial microbubbles, demonstrating the formation of microbubbles with potential for retention in capillary systems.

### EXAMPLE 4

### Microbubble growth by ethanol

The procedure described in Example 2 was repeated using the same microbubble dispersions from Preparation 2 but adding pure ethanol to the edge of the coverglass. The experiment showed growth of microbubbles resulting in microbubbles of larger size than the initial microbubbles, thereby demonstrating the formation of microbubbles with potential for retention in capillary systems.

### EXAMPLE 5

### Microbubble growth by isopropanol

The procedure described in Example 2 was repeated using the same microbubble dispersion from Preparation 2 but adding pure isopropanol to the edge of the coverglass. The experiment showed growth of microbubbles resulting in microbubbles of larger size than the initial microbubbles, thereby demonstrating the formation of microbubbles with potential for retention in capillary systems.

### EXAMPLE 6

### Microbubble growth by acetone

The procedure described in Example 2 was repeated using the same microbubble dispersion from Preparation 2 but adding pure acetone to the edge of the coverglass. The experiment showed growth of microbubbles resulting in microbubbles of larger size than the initial microbubbles, thereby demonstrating the formation of microbubbles with potential for retention in capillary systems.

### EXAMPLE 7

### Microbubble aggregation and growth by a sodium dodecyl sulphate/n-butanol/water microemulsion

One droplet of the microbubble dispersion from Preparation 2, diluted three times with water was placed on an object glass and covered by a coverglass. The sample was placed under a Zeiss Axioskop optical microscope equipped with a 40 x objective and a camera connected to a computer and an image analysis system. A droplet of the sodium dodecyl sulphate/n-butanol/water microemulsion from Preparation 5 was placed on the edge of the coverglass, so that the microemulsion penetrated under the coverglass and into the microbubble dispersion. The behaviour of the microbubble dispersion as the microemulsion front advanced was recorded, showing formation of microbubble aggregates with larger dimensions than the initial microbubbles. A short time afterwards, the microbubbles in the aggregates fused or coalesced to give larger microbubbles, demonstrating the formation of microbubbles with potential for retention in capillary systems.

## Claims

1. A combined preparation for simultaneous, separate or sequential use as an ultrasound contrast agent, said preparation comprising:
i) an injectable aqueous gas dispersion; and
ii) a separately administrable substance or substances capable of destabilising said dispersed gas so as at least transiently to increase the size thereof.

2. A combined preparation as claimed in claim 1
wherein the dispersed gas comprises air, nitrogen, oxygen, carbon dioxide, hydrogen, an inert gas, a sulphur fluoride, selenium hexafluoride, an optionally halogenated silane, an optionally halogenated low molecular weight hydrocarbon, a ketone, an ester or a mixture of any of the foregoing.

3. A combined preparation as claimed in claim 2
wherein the dispersed gas comprises sulphur hexafluoride or a perfluorocarbon.

4. A combined preparation as claimed in claim 3
wherein said perfluorocarbon is perfluoropropane, perfluorobutane or perfluoropentane.

5. A combined preparation as claimed in any of the preceding claims wherein the dispersed gas is stabilised by an initially coalescence-resisting surface membrane, a filmogenic protein, a polymer material, a non-polymeric and non-polymerisable wall-forming material or a surfactant.

6. A combined preparation as claimed in claim 5
wherein said surfactant comprises at least one phospholipid.

7. A combined preparation as claimed in claim 6 wherein at least 75% of said surfactant comprises phospholipid molecules individually bearing net overall charge.

8. A combined preparation as claimed in claim 7 wherein said charged phospholipid molecules are selected from phosphatidylserine, phosphatidylglycerol, phosphatidylinositol, phosphatidic acid and cardiolipin molecules.

9. A combined preparation as claimed in any of the preceding claims comprising one or more destabilising substances which induce growth of the dispersed gas by flocculation, aggregation, agglomeration, coalescence, fusion or Ostwald ripening.

10. A combined preparation as claimed in claim 9 comprising one or more destabilising substances selected from inorganic salts, aliphatic alcohols, aliphatic aldehydes, aliphatic ketones, aliphatic esters, aliphatic ethers, aliphatic amides, aliphatic nitriles, carbohydrates, polyethers, polysaccarides, polyaminoacids, polyvinylpyrrolidone, fatty alcohols, fatty acids, fatty amines, surfactants, steroids, acids, bases and hydrotropes.

11. A combined preparation as claimed in claim 10 comprising one or more destabilising substances selected from calcium chloride, magnesium chloride, ethanol, isopropanol, ethylene glycol, propylene glycol, glycerol, sorbitol, acetaldehyde, acetone, methyl formate, methyl acetate, propyl formate, ethyl acetate, ethyl methyl ether, methyl propyl ether, di-isopropyl ether, N,N-dimethylformamide, N,N-dimethylacetamide, acetonitrile, glucose, sucrose, polyethylene glycol, polypropylene glycol, polyoxyethylene-polyoxypropylene block copolymers, dextran, starches, polylysine, gelatin, cholesterol, and surface active alkyl carboxylates, alkyl sulphonates, alkyl sulphates, dialkyl sulphosuccinates, alkyl pyridinium salts, alkylammonium salts, alkyl polyethylene glycol ethers, alkyl polyethylene glycol esters and sorbitol fatty acid esters.

12. A combined preparation as claimed in any of the preceding claims which further includes a vasodilator drug.

13. A combined preparation as claimed in claim 12
wherein said vasodilator drug is adenosine.

14. A combined preparation as claimed in any of claims 1 to 11 which further includes a therapeutic drug.

15. A combined preparation as claimed in any of claims 1 to 11 which further includes contrast-enhancing moieties for an imaging modality other than ultrasound.

## Patentansprüche

1. Kombiniertes Präparat für den simultanen, separaten oder aufeinanderfolgenden Gebrauch als Ultraschallkontrastmittel, wobei dieses Präparat aufweist:
i) eine injizierbare, wäßrige Gasdispersion und
ii) eine separat verabreichbare Substanz, oder Substanzen, mit der Fähigkeit, das dispergierte Gas zu entstabilisieren, um zumindest vorübergehend seine Größe zu erhöhen.

2. Kombiniertes Präparat gemäß Anspruch 1, wobei das dispergierte Gas Luft, Stickstoff, Sauerstoff, Kohlendioxyd, Wasserstoff, ein Inertgas, ein Schwefelfluorid, Selenhexafluorid, ein gegebenenfalls halogeniertes Silan, einen gegebenenfalls halogenierten Kohlenwasserstoff mit niederem Molekulargewicht, ein Keton, einen Ester oder eine Mischung aus beliebigen der vorgenannten Stoffe umfasst.

3. Kombiniertes Präparat gemäß Anspruch 2, wobei das dispergierte Gas Schwefelhexafluorid oder einen Perfluorkohlenstoff umfasst.

4. Kombiniertes Präparat gemäß Anspruch 3, wobei der Perfluorkohlenstoff Perfluorpropan, Perfluorbutan oder Perfluorpentan ist.

5. Kombiniertes Präparat gemäß einem der vorhergehenden Ansprüche, wobei das dispergierte Gas durch eine anfänglich koaleszensbeständige Oberflächenmembran, ein filmbildendes Protein, ein Polymermaterial, ein nicht-polymeres und nicht polymerisierbares Wandbildungsmaterial oder ein Tensid stabilisiert wird.

6. Kombiniertes Präparat gemäß Anspruch 5, wobei das Tensid mindestens ein Phospholipid aufweist.

7. Kombiniertes Präparat gemäß Anspruch 6, wobei mindestens 75% des Tensids Phospholipidmoleküle umfassen, welche einzeln die gesamte Nettoaufladung tragen.

8. Kombiniertes Präparat gemäß Anspruch 7, wobei die geladenen Phospholipidmoleküle unter Phosphatidylserin, Phosphatidylglycerol, Phosphatidylinositol, Phosphatidsäure und Cardiolipin-Molekülen ausgewählt sind.

9. Kombiniertes Präparat gemäß einem der vorhergehenden Ansprüche, das eine oder mehrere entstabilisierende Substanzen enthält, die das Wachstum des dispergierten Gases durch Flockenbildung, Aggregation, Agglomeration, Koaleszenz, Fusion oder Ostwald-Reifung auslösen.

10. Kombiniertes Präparat gemäß Anspruch 9, das eine oder mehrere entstabilisierende Substanzen enthält, die unter anorganischen Salzen, aliphatischen Alkoholen, aliphatischen Aldehyden, aliphatischen Ketonen, aliphatischen Estern, aliphatischen Ethern, aliphatischen Amiden, aliphatischen Nitrilen, Kohlenhydraten, Polyethern, Polysachariden, Polyaminosäuren, Polyvinylpyrrolidon, Fettalkoholen, Fettsäuren, Fettsäureaminen, Tensiden, Steroiden, Säuren, Basen und hydrotropen Stoffen ausgewählt sind.

11. Kombiniertes Präparat gemäß Anspruch 10, das eine oder mehrere entstabilisierende Substanzen enthält, die unter Calciumchlorid, Magnesiumchlorid, Ethanol, Isopropanol, Ethylenglycol, Propylenglycol, Glycerol, Sorbitol, Acetaldehyd, Aceton, Methylformiat, Methylacetat, Propylformiat, Ethylacetat, Ethylmethylether, Methylpropylether, Diisopropylether, N, N-Dimethylformamid, N, N-Dimethylacetamid, Acetonitril, Glucose, Saccharose, Polyethylenglycol, Polypropylenglycol, Polyoxethylen-Polyoxypropylen-Blockcopolymeren, Dextran, Stärken, Polylysin, Gelatine, Cholesterol und oberflächenaktiven Alkylcarboxylaten, Alkylsulfonaten, Alkylsulfaten, Dialkylsulfosuccinaten, Alkylpyridinium-Salzen, Alkylammonium-Salzen, Alkylpolyethylenglycolethern, Alkylpolyethylenglycolestern und Sorbitolfettsäureestern ausgewählt sind.

12. Kombiniertes Präparat gemäß einem der vorhergehenden Ansprüche, das ferner eine gefäßerweiternde Droge miteinschließt.

13. Kombiniertes Präparat gemäß Anspruch 12, wobei die gefäßerweiternde Droge Adenosin ist.

14. Kombiniertes Präparat gemäß einem der Ansprüche 1 bis 11, das ferner eine therapeutische Droge miteinschließt.

15. Kombiniertes Präparat gemäß einem der Ansprüche 1 bis 11, das weiterhin kontrastfördernde Anteile für eine von der Ultraschallbildgebung unterschiedliche Bildgebungsmodalität enthält.

## Revendications

1. Une préparation combinée pour une utilisation simultanée, séparée ou séquentielle comme agent de contraste ultrasonore, ladite préparation comprenant:
i) une dispersion aqueuse de gaz susceptible d'être injectée ; et
ii) une substance susceptible d'être administrée séparément ou des substances capables de déstabiliser ledit gaz dispersé de manière à augmenter au moins de façon transitoire son taux.

2. Une préparation combinée comme revendiquée dans la revendication 1 dans laquelle le gaz dispersé comprend de l'air, du nitrogène, de l'oxygène, du dioxyde de carbone, de l'hydrogène, un gaz inerte, un fluorure de soufre, de l'hexafluorure de sélénium, un silane halogéné de façon optionnelle, un hydrocarbure à faible poids moléculaire, halogéné de façon optionnelle, une cétone, un ester ou un mélange de n'importe laquelle des substances citées ci-dessus.

3. Une préparation combinée comme revendiquée dans la revendication 2, dans laquelle le gaz dispersé comprend de l'hexafluorure de soufre ou un hydrocarbure perfluoré.

4. Une préparation combinée comme revendiquée dans la revendication 3, dans laquelle ledit hydrocarbure perfluoré est du perfluoropropane, du perfluorobutane ou du perfluoropentane.

5. Une préparation combinée comme revendiquée dans n'importe laquelle des revendications précédentes dans laquelle le gaz dispersé est stabilisé par une membrane de surface résistant initialement à la coalescence, une protéine filmogénique, un matériau polymère, un matériau non-polymérique et non polymerisable formant une paroi ou un agent de surface.

6. Une préparation combinée comme revendiquée dans la revendication 5, dans laquelle ledit agent de surface comprend au moins un phospholipide.

7. Une préparation combinée comme revendiquée dans la revendication 6 dans laquelle au moins 75% dudit agent de surface comprend des molécules de phospholipides portant de façon individuelle une charge totale nette.

8. Une préparation combinée comme revendiquée dans la revendication 7 dans laquelle lesdites molécules phospholipides chargées sont choisies à partir de molécules de phosphatidylsérine, phosphatidylglycerole, phosphatidylinositole, acide phosphatidique et de cardiolipine.

9. Une préparation combinée comme revendiquée dans n'importe laquelle des revendications précédentes comprenant une ou plusieurs substances déstabilisantes qui génèrent la croissance du gaz dispersé par floculation, agrégation, agglomération, coalescence, fusion ou maturation d'Ostwald.

10. Une préparation combinée comme revendiquée dans la revendication 9 comprenant une ou plusieurs substances déstabilisante choisie(s) à partir de sels inorganiques, d'alcools aliphatiques, d'aldéhydes aliphatiques, de cétones aliphatiques, d'esters aliphatiques, d'éthers aliphatiques, d'amides aliphatiques, de nitriles aliphatiques, d'hydrates de carbone, de polyéthers, de polysaccharides, de polyamino -acides, de polyvinylpyrrolidone, d'alcools gras , d'acides gras , d'amines grasses, d'agents de surface, de stéroïdes, d'acides, de bases et d'hydrotropes.

11. Une préparation combinée comme revendiquée dans la revendication 10 comprenant une ou plusieurs substances déstabilisantes choisie(s) à partir du chlorure de calcium, du chlorure de magnésium, de l'éthanol, de l'isopropanole, de l'éthylène glycol, du propylène glycol, du glycérol, du sorbitol, de l'acetaldéhyde, de l'acétone, du formiate de méthyle, de l'acétate de méthyle, du formiate de propyle, de l'acétate d'éthyle, de l'éther de méthyle éthyle, de l'éther de propyle méthyle, de l'éther di-isopropyle, du N, N-diméthylformamide, du N,N-dimethylacétamide, de l'acétonitrile, du glucose, du sucrose, du glycol polyéthylène, du glycol polypropylène, des copolymères de blocs de polyoxyéthylène-polyoxypropylène, du dextrane, des amidons, de la polylysine, de la gélatine, du cholestérol, et des carboxylates d'alkyle tensioactifs, des sulfonâtes d'alkyle, des sulfates d'alkyle, des sulphosuccinates de dialkyle, des sels d'alkylpyrimidine, des sels d'alkyl ammonium, des éthers d'alkyle polyéthylène glycol, des esters d'alkyle polyéthylène glycol et des esters d'acides gras de sorbitol.

12. Une préparation combinée comme revendiquée dans l'une quelconque des revendications précédentes qui comprend en outre un médicament vasodilatateur.

13. Une préparation combinée comme revendiquée dans la revendication 12, dans laquelle le médicament vasodilatateur est de l'adénosine.

14. Une préparation combinée comme revendiquée dans l'une quelconque des revendications 1 à 11 qui comprend en outre un médicament thérapeutique.

15. Une préparation combinée comme revendiquée dans l'une quelconque des revendications 1 à 11 qui comprend en outre des éléments augmentant le contraste autre que les ultrasons pour un mode d'application de formation d'image.
